# EUROPEAN PATENT SPECIFICATION

(11) **EP 3 453 335 B1**
(45) Date of publication and mention of the grant of the patent: **19.05.2021**
(21) Application number: 18189282.9
(22) Date of filing: 25.08.2009
(51) Int. Cl.: A61B 8/08, A61B 8/12, A61B 8/00, A61B 18/14, A61B 90/00

(54) **ABLATION DEVICE WITH ARTICULATED IMAGING TRANSDUCER**
ABLATIONSVORRICHTUNG MIT GELENKIGEM BILDGEBUNGSWANDLER
DISPOSITIF D'ABLATION DOTÉ D'UN TRANSDUCTEUR D'IMAGERIE ARTICULÉ

(30) Priority: 26.08.2008 US 198861
(43) Date of publication of application: 13.03.2019
(62) Divisional of application: 09812027.2
(73) Proprietor: Gynesonics, Inc., Redwood City, CA 94063 (US)
(72) Inventor: DECKMAN, Robert, K, San Bruno, CA California 94066 (US); PLACEK, Brian, Menlo Park, CA California 94025 (US); MUNROW, Michael, A, Belmont, CA California 94002 (US); GERBI, Craig, Half Moon Bay, CA California 94019 (US)
(74) Representative: Clark, Jane Anne

(56) References cited:
- US-A- 5 469 853
- US-A- 5 800 378
- US-A- 6 080 150
- US-A1- 2006 058 680
- US-A1- 2007 203 486
- US-A1- 2007 249 939

## Description

### BACKGROUND OF THE INVENTION

1. Field of the Invention. The present invention relates generally to medical devices and methods. More particularly, the present invention relates to an imaging and therapy device having a deployable treatment needle or needles and a pivotal imaging array.

Uterine fibroids are benign tumors in the uterine wall and are the most common tumor of the female pelvis. Fibroids afflict up to 30% of women of childbearing age and can cause significant symptoms including discomfort, pelvic pain, mennorhagia (excessive bleeding), anemia, infertility, and miscarriage. While fibroids may be located in the muscle (intramural), adjacent to the endometrium (submucosal), or in the outer layer of the uterus (subserosal), and can grow up to several centimeters in diameter.

Current treatments for fibroids include both pharmaceutical and surgical intervention. Pharmaceutical treatments include the administration of NSAIDS, estrogen-progesterone combinations, and the like. Medications, however, are generally ineffective and are palliative rather than curative. Surgical interventions include myomectomy, where fibroids are removed in an open surgical procedure requiring laparotomy and general anesthesia, and hysterectomy, involving complete surgical removal of the uterus. Both these procedures are long and have significant blood loss.

As improvements over open surgical procedures, several minimally invasive procedures have been developed. Laparoscopic myomectomy is a laparoscopic procedure requiring highly skilled laparoscopic gynecologists. Uterine artery embolization relies on blocking the uterine artery supplying blood to the fibroid by injecting small particles. While sometimes effective, common complications of arterial embolization include infection, premature menopause, and severe pelvic pain. A third approach relies on complete endometrial ablation, which is generally effective for treating bleeding but less reliable for treating fibroids.

More recently, and of particular interest to the present invention, the use of radio frequency needles and other ablation elements for treating individual fibroids via a transvaginal approach has been proposed. As described, for example, in published U.S. Patent Applications 2006/0189972; 2007/0179380; 2007/0249936; and 2008/0033493, each of which is commonly assigned with the present application, a probe carrying a curved needle is used to treat individual fibroids. The probe carries on-board ultrasonic or other imaging so that the needle can be guided into the fibroid under direct observation. While highly effective in many cases, accurate advancement of a curved needle into a fibroid can be problematic. Moreover, use of a single needle does not always deliver sufficient energy to fully ablate relatively large fibroids.

For these reasons, it would be desirable to provide alternative devices and methods for treating, ablating, or removing uterine fibroids and other tissue masses. It would be particularly desirable if such methods and devices were able to treat uterine fibroids which are large, difficult to penetrate, or which otherwise resist treatment with curved and laterally deployed needles. At least some of these objectives will be met by the inventions described below.

2. Brief Description of the Background Art. The following US published applications discussed above are relevant to the present invention: 2006/0189972; 2007/0179380; 200710249936; and 2008/0033493.

US 2007/0249939 describes a delivery system including rigid shaft, an imaging core, and an interventional core. The rigid delivery shaft has a proximal end, an angled distal tip, and an axial passage therethrough. The imaging core comprises an ultrasound imaging insert disposed within the axial passage. The imaging insert has an ultrasound array within a distal portion thereof, wherein the ultrasound array is tilted relative to a shaft axis. The interventional core comprises a curved ablation needle coupled to the rigid shaft. An angle of needle curvature may be inversely proportional to the ultrasound array tilt and tip angle
US5469853 describes a bendable ultrasonic probe that bends against a longitudinal direction of the carrier. The bendable portion bends in a direction away from an exit port of a therapy channel enabling an ultrasonic device carried by the bendable portion to be repositioned relative to the therapy channel and the exit port. An endosurgical tool may exit the exit port.

US2006/0058680 describes a guide device for a laparoscopic ultrasound probe. The guide device includes a sheath that is adapted to fit over at least a portion of the ultrasound probe, and a guide coupled to the sheath for guiding a medical tool inserted therethrough to position the medical tool in a plane of an ultrasound image obtained via the ultrasound probe. The laparoscopic ultrasound probe is flexible so that the distal end thereof can be controlled to bend.

### BRIEF SUMMARY OF THE INVENTION

The present invention is set out in the appended claims. Described herein are apparatus for imaging and treating fibroids and other tumors and tissue mases located in the walls of a uterus or other body cavity. The apparatus and systems comprise a straight shaft having a distal end and a proximal end. A delivery needle, preferably straight, is reciprocatably coupled to the shaft, typically being mounted in a straight lumen in the shaft, so that a tissue-penetrating tip of the needle can be distally advanced from the shaft along an axial path. The delivery needle carries tines forming a needle array, deployable from within the delivery needle. A tip or other structure is pivotally attached to the distal end of the shaft and is moveable between a position parallel to the axial path and a position at an acute or right angle relative to the axial path. The pivotable tip carries or comprises an ultrasonic imaging array, and the tip can be oriented to align a field of view of the imaging array with the needle as the needle is advanced along the axial path.

The combination of a straight shaft, delivery needle, and pivotally attached tip or imaging array has a number of advantages. The straight shaft and needle can be advanced with precision into tissue surrounding the body cavity, where the needle can be made sufficiently strong to resist unwanted deflection of the type which could occur with other needle configurations. The use of a delivery needle and shaft also enables and facilitates the deployment of a needle array, including a plurality of tines, from the delivery needle to increase the volume of tissue being treated with the needle array. The pivotable imaging array allows straightening of the imaging array to provide a low profile for introduction through the cervix into the uterus, while also allowing reorientation to cover a wide range of viewing fields after entering the uterus or other body cavity to permit locating fibroids and other tumors and to further follow the advance of the needle array into the fibroids or other tumors. It should be noted that in the preferred embodiment, the delivery needle is for delivery only, and does not provide treatment. In alternative embodiments, the delivery needle may be used for treatment. The pivotable tip further allows the effective field of view of the ultrasound image to be increased by pivoting the tip, which has the effect of sweeping the ultrasound image. The tip may be pivoted to enhance the view of the delivery needle and/or the needle array, including tines.

In the preferred embodiment, the imaging array will be formed on an imaging core, where the imaging core is removably positionable in the straight shaft so that the imaging array extends into the pivotally attached tip. The straight shaft will usually be rigid while the imaging core is relatively flexible, allowing the imaging core to bend at the point where the tip is pivotally attached to the shaft. In alternate embodiments, the needle assembly may be attached directly to the ultrasound probe or the imaging core may be hinged at the point where the tip is pivotally attached to the shaft.

The delivery needle will carry a needle array having at least one tine which can be advanced from the delivery needle, usually carrying a plurality of tines, where the tines are reciprocatably attached to the delivery needle to permit deployment and retraction, usually after the delivery needle has been advanced into target tissue. A plurality of tines will usually be arranged to radially diverge from the delivery needle as the tines are distally advanced. Optionally, at least one additional tine may be reciprocatably mounted on the delivery needle in a range to be advanced axially from the needle, often forming a center axis to a symmetric deployment of radially diverging tines. In order to localize the treatment, the tines may be electrically conductive while the delivery needle itself is electrically non-conductive or insulating. In such cases, the tines may be arranged to be connected to a single pole of an electrosurgical power supply in order to provide for monopolar treatment. Alternatively, a certain number of the tines may be adopted to one pole of the power supply while others are connected to the other pole, providing for bipolar treatment.

In certain exemplary embodiments, the imaging and therapeutic delivery system will further comprise a handle attached to the proximal end of the straight shaft. The handle may include a lever coupled to the pivotally attached distal tip by one or more pull rods. The lever can be pulled or pushed to actuate the pull rod(s) to pivot the tip. Additionally, the handle may include a first slide mechanism coupled to the delivery needle, where the slide mechanism can be reciprocated to advance and retract the needle along the axial path. In the embodiments which include the plurality of tines, the tines may be reciprocatably attached to the delivery needle and connected to a second slide mechanism on the handle, optionally being disposed on the first slide mechanism itself, to advance and retract the tines relative to the needle. Optionally, a stop structure may be disposed on the pivotally attached tip so that the stop structure prevents advancement of the needle when the tip is parallel to the axial path of the needle.

Also disclosed are methods for treating uterine fibroids. The methods include introducing a straight shaft into the uterus. Uterine fibroids are then located using an ultrasonic imaging transducer carried by or formed as part of a pivotable tip attached to a distal end of the shaft. The tip is pivoted to reposition a field of view of the ultrasonic transducer carried by the tip. Optionally, the tip may block advancement of the needle when disposed parallel to the shaft (prior to deployment) and allow advancement when pivoted from the parallel orientation. A delivery needle may be axially advanced from the distal tip of the shaft into tissue near or in a uterine fibroid located using the ultrasonic transducer. Advancement of the needle may be observed by the transducer by aligning the field of view with the needle advancement.

In preferred aspects of the methods, the shaft is introduced to the uterus via a transvaginal and transcervical introduction. Locating fibroids may comprise manually rotating and translating the shaft to scan the uterine wall with the ultrasonic transducer. Locating may also comprise pivoting the ultrasonic transducer to adjust the field of view. Optionally, an array including a plurality of tines may be advanced from the delivery needle after the needle has been advanced into tissue at or near the uterine fibroid. This method will sweep the ultrasound field of view relative to the needle and anatomy to be imaged. The fibroid is then treated by delivering energy from the needle and/or tines into the fibroid, typically radiofrequency energy, including both monopolar and bipolar radiofrequency energy. Usually, the tines will be electrically active to deliver the radiofrequency energy while the delivery needle is electrically non-conductive to limit the distribution of energy in the uterine wall or other tissue being treated.

### BRIEF DESCRIPTION OF THE DRAWINGS

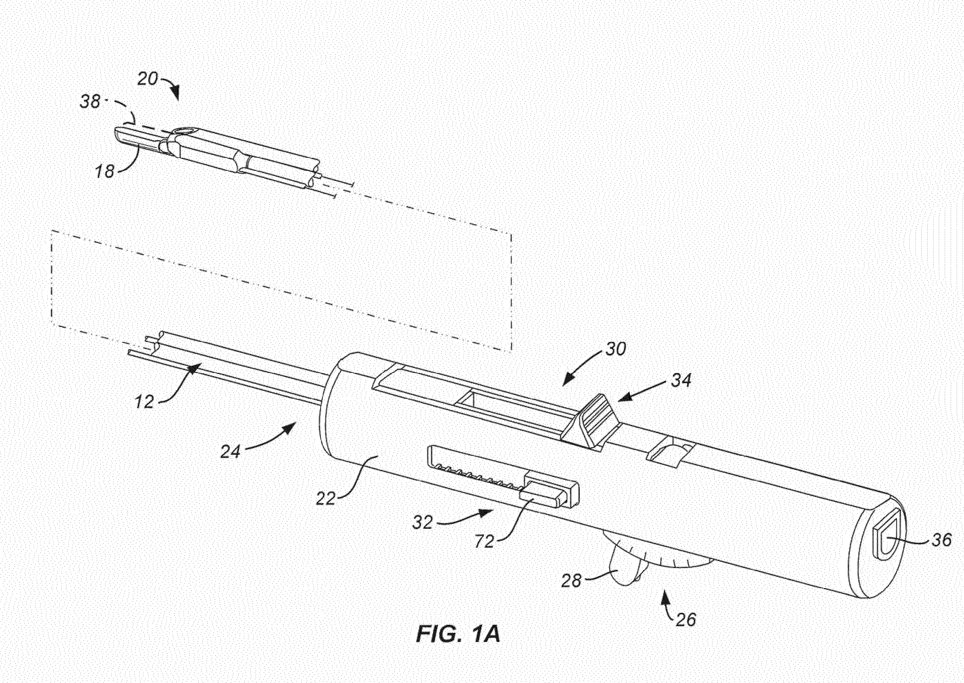

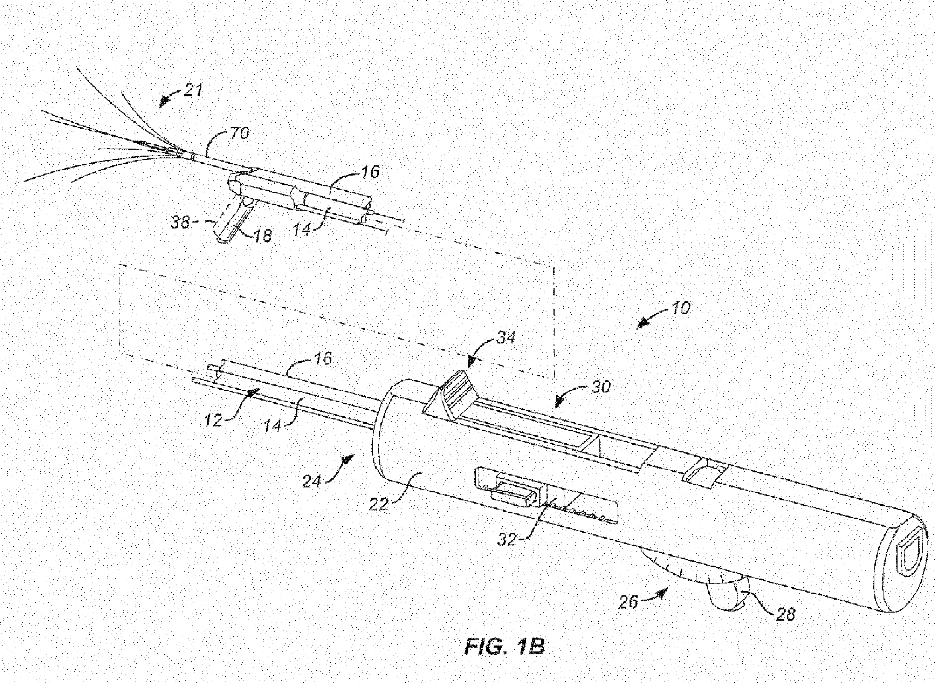

Figs. 1A and 1B are perspective views of an imaging and therapeutic delivery system constructed in accordance with the principles of the present invention shown with portions broken away. In Fig. 1A, a delivery needle and array including radially diverging tines are retracted within the shaft of the device, and a pivotally attached tip is shown in axial alignment with the axial deployment path of the needle. In Fig. 1B, the delivery needle and associated tines are shown in their deployed configuration with the pivotally attached tip shown oriented at an acute angle relative to the axial advancement path of the needle.
Fig. 2 illustrates the imaging and therapeutic delivery system of Figs. 1A and 1B in cross-section. Fig. 2A is a detail of the distal tip of the device illustrated in Fig. 2. Figs. 2B and 2C illustrate a stop structure on the pivotally attached tip which prevents needle advancement prior to deployment of the tip.
Figs. 3A and 3B illustrate the pivotal tip deployment mechanism in detail, also in cross-section.
Figs. 4A-4C illustrate the relative movement of the deployment mechanism and the pivotal tip, as the deployment mechanism is actuated.
Figs. 5 and 6 are side and top views of the imaging and therapeutic delivery system shown with portions broken away in a non-deployed configuration.
Figs. 7 and 8 are views similar to Figs. 5 and 6, except that the delivery needle has been deployed and the pivotally attached tip has been positioned at an acute angle.
Figs. 9 and 10 are views similar to Figs. 5 and 6 and Figs. 7 and 8, respectively, further illustrating the deployment of the needle array, comprising radially diverging tines from the delivery needle.
Figs. 11A and 11B illustrate deployment of the delivery needle and tines into tissue.

### DETAILED DESCRIPTION OF THE INVENTION

Referring to Figs. 1A and 1B, an imaging and therapeutic delivery system constructed in accordance with the principles of the present invention comprises a straight shaft assembly 12 including a hollow rod 14 and a needle tube 16. A tip 18 which is adapted to receive an ultrasonic imaging array (shown in broken line at 38) is pivotally attached to a distal end 20 of the hollow rod 14 of the straight shaft assembly 12. A needle and tine array 21 (Fig. 1B) is deployed through a lumen or central passage in the needle tube 16 at a distal end 20 of the shaft assembly 12. A handle assembly 22 is attached to a proximal end 24 of the straight shaft assembly 12 and includes a pivoting mechanism 26, typically found on its lower surface as illustrated, for selectively pivoting the imaging array tip 18 between a low profile configuration where the tip 18 is axially aligned with the axis of the shaft assembly 12, as illustrated in Fig. 1A, and a deflected configuration where the tip 18 is oriented at an acute or right angle relative to the axis of the shaft, as illustrated in Fig. 1B. The tip 18 may be placed in its axially aligned, low profile configuration for introduction to the body cavity, for example through the cervix into the uterus, and may be shifted to its deflected configuration in order to image tissue and/or to track deployment of the needle/tine array 21. As described in more detail below, the pivoting mechanism 26 includes a lever 28 which may be manually retracted from the distally advanced configuration shown in Fig. 1A to the proximally retracted configuration shown in Fig. 1B in order to pivot the tip 18.

The handle 22 will also include a delivery needle/tine deployment mechanism 30 which includes a first slide subassembly 32 and a second slide subassembly 34. The handle will usually further include a port 36 at its proximal end. Port 36 allows introduction of an ultrasonic or other imaging core, where the imaging core has an imaging array 38, typically an ultrasonic imaging array as described in detail in copending Application Nos. 11/620,594; 11/620,569; and 11/564,164, published as US2007/017938, US2007/0161905 and US2007/0249936 respectively. The proximal end of the handle will also allow electrical connections to be made to the needle/tine array. Additionally, the distal end of the handle will provide a standard luer connection for the infusion of non-conductive coupling fluids.

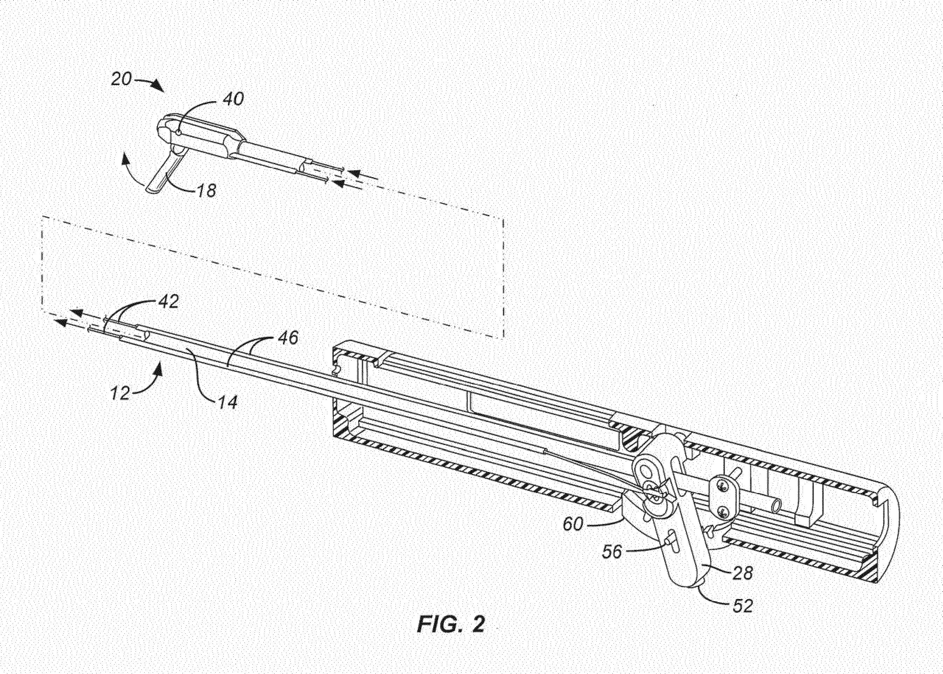

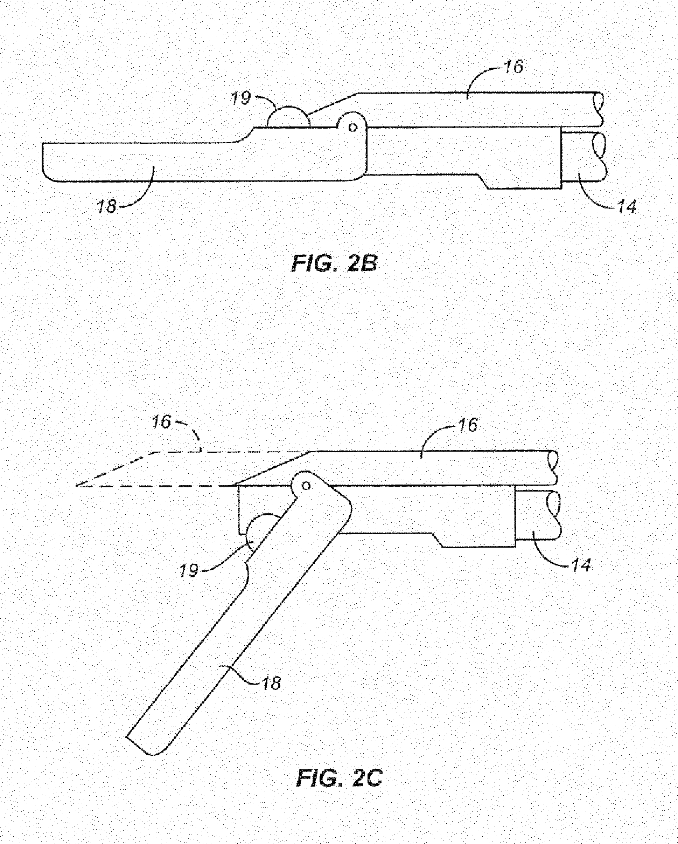

A stop structure 19 is attached to an upper surface of the pivotally attached tip 18, as illustrated in Figs. 2B and 2C. When the tip 18 is parallel to the axis of the shaft (hollow rod 14), the stop structure 19 will block the advancement path of the needle 16 (as shown in Fig. 2B). This is advantageous since it prevents accidental needle advancement while the shaft assembly 12 is in the introductory configuration. Deployment of the tip 18, as shown in Fig. 2C, moves the stop structure 19 out of the advancement path of the needle 16, as described below.

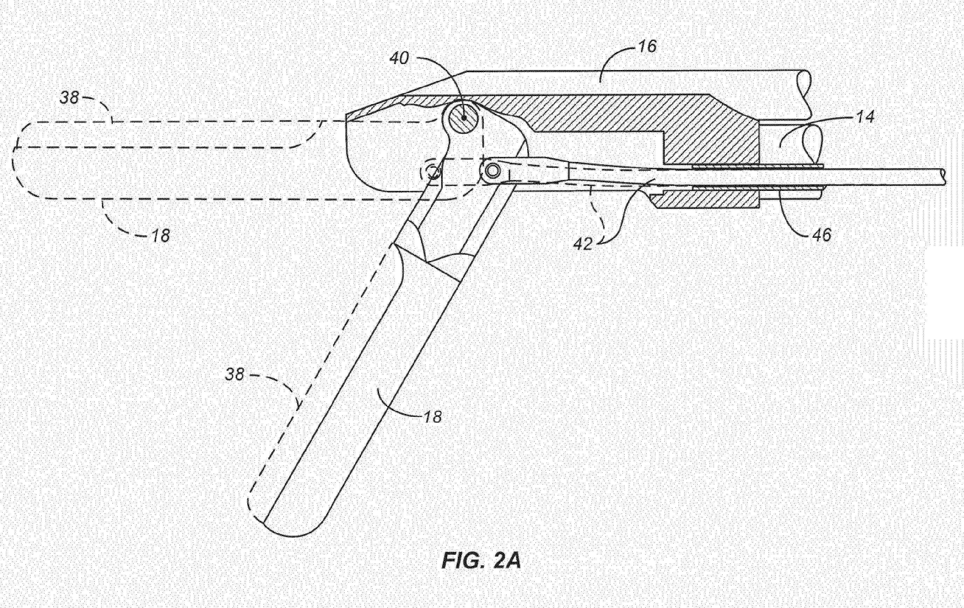

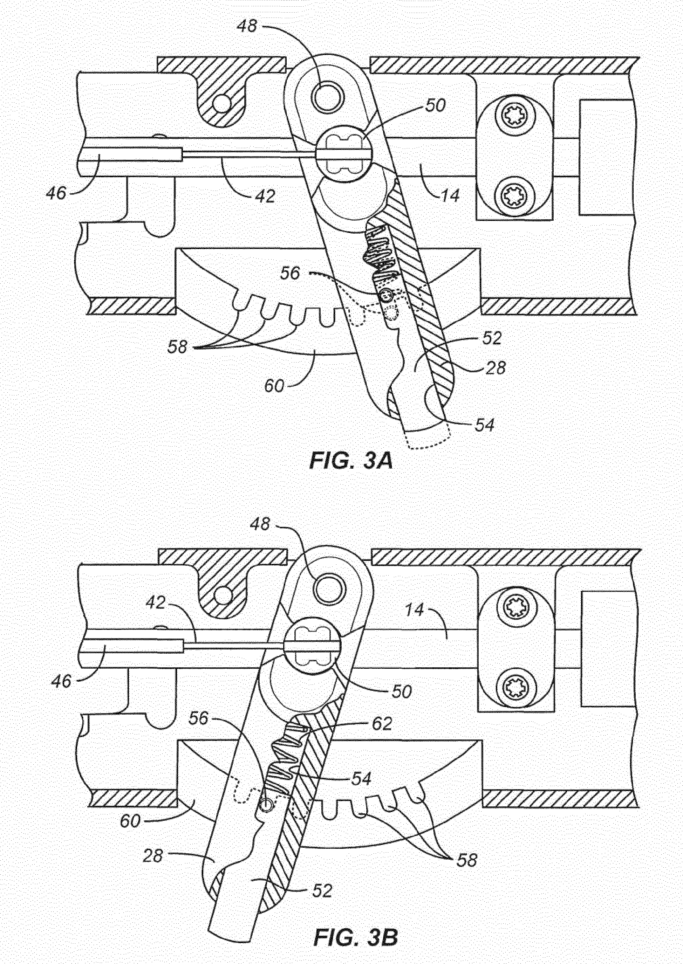

Referring now to Figs. 2, 2A, 3A, and 3B, operation of the pivot mechanism 26 for selectively deflecting the tip 18 disposed at the distal end of the straight shaft assembly 12 will be described. For clarity, components of the first slide assembly 32 and second slide assembly 34 have been removed from the view in Fig. 2. The tip 18 is pivotally attached at the distal end 20 of the straight shaft assembly 12 by a pivot pin 40 or similar structure, as best seen in Fig. 2A. A pair of pull rods 42 are attached at anchors 44 so that drawing the wires in a proximal direction will deflect the tip 18 from an axially aligned configuration, as shown in broken line in Fig. 2A, to the deflected configuration, as shown in full line in Fig. 2A. The rods 42 extend through tubes 46 disposed on each side of the hollow rod 14 of the shaft assembly 12. As best seen in Figs. 3A and 3B, the rods 42 are attached at their proximal ends to a rotating anchor 50 disposed in lever 28. Thus, by drawing the lever 28 proximally, as shown in Fig. 3A, the tip 18 may be laterally deflected, as shown in full line in Fig. 2A. Conversely, by pushing the lever 28 in a distal direction, as shown in Fig. 3B, the tip 18 may be returned to the axially aligned configuration as shown in broken line in Fig. 2A. The lever 28 is pivotally attached to the body of handle 22 by a pivot pin 48 so that the anchor 50 is offset from the point of rotation of the lever 28. Thus, the anchor 50 is actually translated as the lever is rotated back and forth about the pivot pin 48.

A locking pin 52 allows the lever 28 to be selectively locked in place to hold the pivot tip 18 in a fixed orientation. Locking pin 52 is mounted in a central passage 54 of the lever 28 and carries a pin 56 which seats in one of a plurality of pockets 58 formed in an arcurate locking strip 60. Thus, the lever 28 can be released by pressing the pin 52 against spring 62 so that the pin 56 is lifted out of the pocket 58, as shown in Fig. 3A. In this configuration, the lever may be moved freely back and forth to deploy the tip 18. When the tip 18 is in its desired location, the locking pin 52 may be released to permit pin 56 to engage the closest pocket 58 where it is held in place by spring 62. It will be appreciated that the lever 28 will typically be advanced forwardly to close the tip 18 to a low profile configuration for introducing the imaging and therapy delivery system 10 to the patient for treatment, for example through the cervix into the uterus. Once in place, the lever 28 can be unlocked using the locking pin 52 and oriented to a desired angle relative to the shaft assembly 12 to permit imaging and, in particular, to allow advancement of the delivery needle 70 in the tissue to be observed.

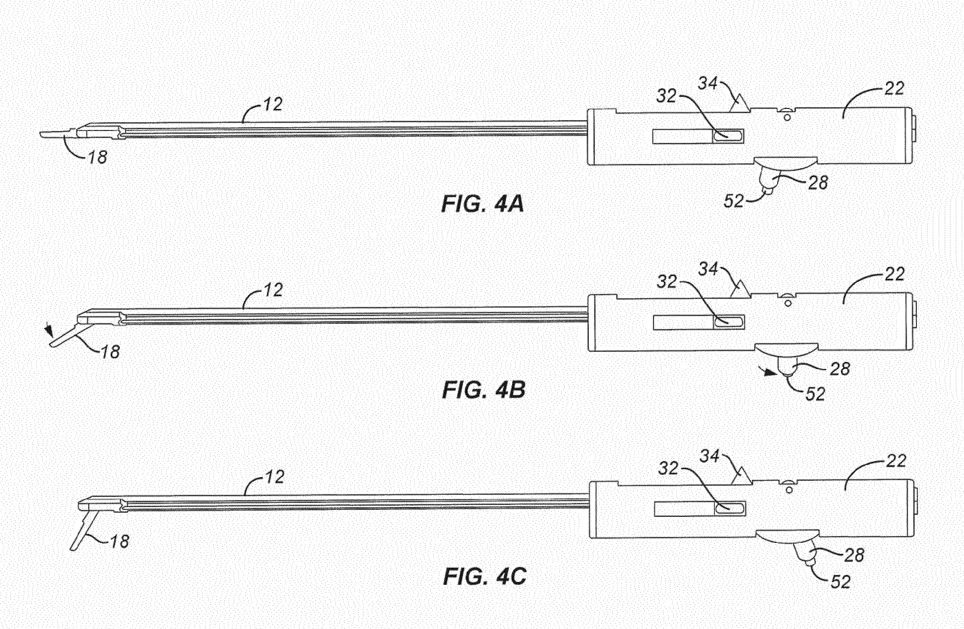

Referring now to Figs. 4A-4C, use of the lever 28 for deflecting the tip 18 is illustrated. Initially, the tip 18 is axially aligned with the axis of the shaft assembly 12 and the lever 28 is in its forward or distal-most position, as shown in Fig. 4A. By depressing locking pin 52, as shown in Fig. 4B, lever 28 may be drawn proximally as indicated by the adjacent arrow, to deflect the tip 18 away from the axis of shaft 12, as shown by the arrow adjacent the tip in Fig. 4B. When the lever 28 reaches its fully proximal position, as shown in Fig. 4C, the tip 18 has been fully deflected away from the axis of shaft assembly 12. Note that slide subassemblies 32 and 34 (for extending delivery needle 70 and needle array 21) have not been activated in Figs. 4A-4B.

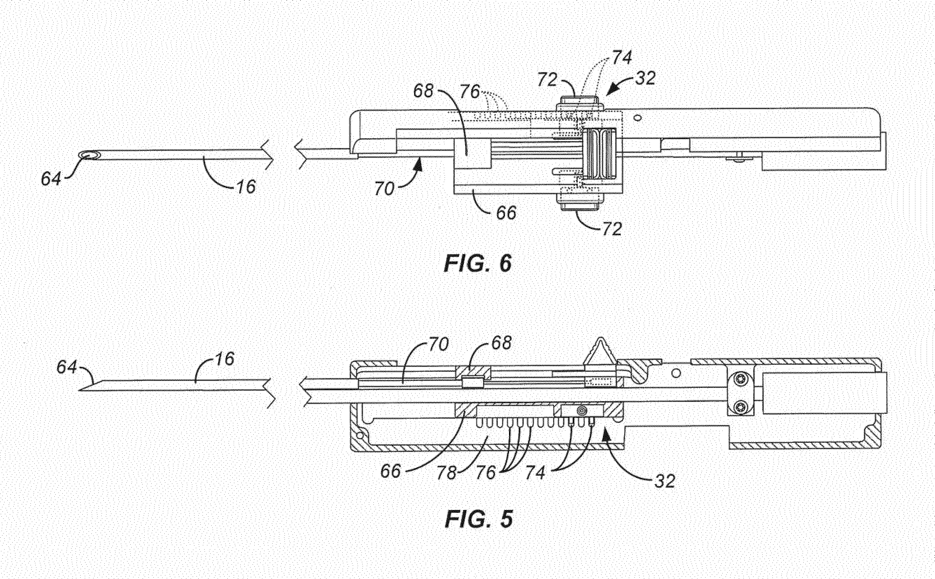

Referring now to Figs. 5-10, operation of the first slide subassembly 32 and the second slide subassembly 34 will be described. For clarity, portions of the pivot mechanism 26 have been removed from these views. Prior to deployment, as shown in Figs. 5 and 6, the needle/tine array 21 is fully drawn into the central passage of needle tube 16. Needle tube 16 has an open distal tip 64 through which the delivery needle and tines will emerge when advanced using the slide subassemblies 32 and 34.

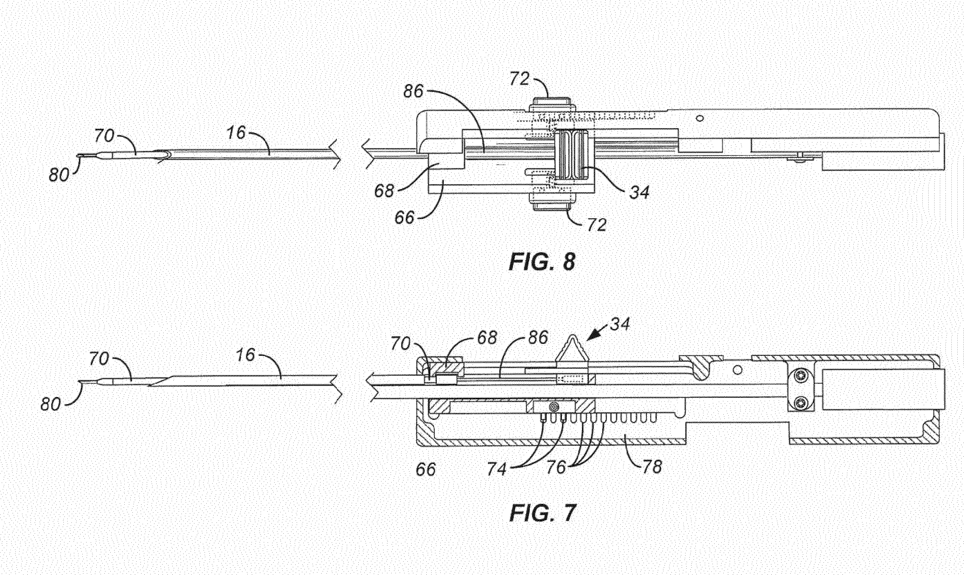

The first slide subassembly 32 comprises a reciprocating carriage 66 having a coupling 68 attached to a proximal end of the needle 70. The carriage 66 may be axially advanced and retracted by manually pressing buttons 72 to disengage pins 74 (Fig. 5) from pockets 76 in a straight locking strip 78. Once the pins 74 are disengaged, the carriage 66 may be distally advanced, as shown in Figs. 7 and 8, to advance tip 80 of needle 70 from the distal end of the needle tube 16. The buttons 72 may then be released to allow pins 74 to reenter the adjacent pockets 76 in the locking strip 78, thus locking the needle 70 in place.

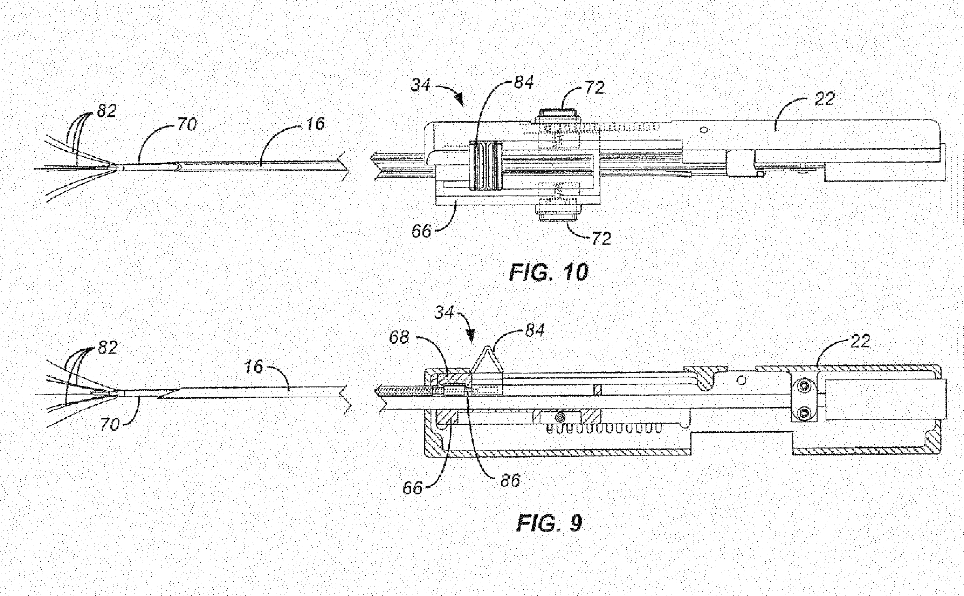

Referring now in particular to Figs. 9 and 10, a plurality of radially diverging tines 82 may be deployed from the distal end of needle 70 using the second slide subassembly 34 which includes a thumb slide 84. The thumb slide 84 is reciprocatably carried in the carriage 66 so that the thumb slide will advance the tines relative to the needle. The thumb slide is connected to a tine rod 86 which enters a hollow central passage or lumen of the needle 70 and is coupled to the plurality of tines 82 so that advancement of the thumb slide 84 from the retracted position shown in Figs. 7 and 8 to the distally advanced position shown in Figs. 9 and 10 causes the tines 82 to emerge from the distal end of the needle 70. The tines 82 are preferably formed from a straight, resilient metal, such as stainless steel, nickel titanium, or the like, and are deflected outwardly by ramps (not shown) in the distal end of the needle. Optionally, a lockout circuit (not shown) may be provided to prevent energizing the tines if the tines are not fully advanced.

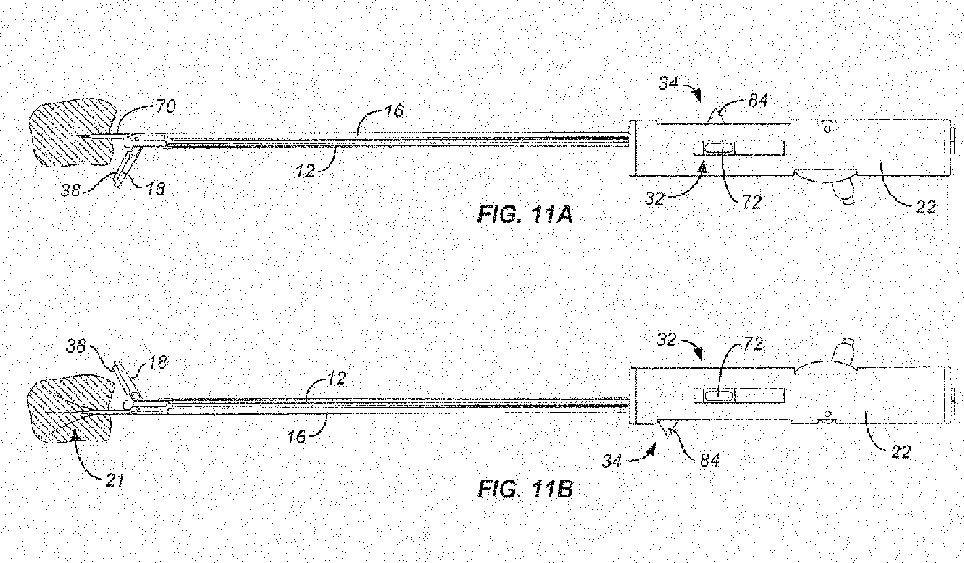

The use of the imaging and therapeutic delivery system 10 of the present invention is illustrated in Figs. 11A and 11B. After imaging using the imaging array 38 carried on or in tip 18, the needle 70 is advanced into target tissue identified by the imaging using the first slide subassembly 32, as shown in Fig. 11A. Usually, the position of the tip 18 will be adjusted to assure that travel of the needle 70 into the tissue may be observed. After the location of the needle tip 80 has been confirmed, the thumb slide 84 of the second slide subassembly 34 may then be advanced, as shown in Fig. 11B, to extend the tines 82 into the tissue. In the preferred embodiments of the present invention, the needle 70 and tines 82 will be rotatably connected to the remainder of the device to allow the handle to be rotated, thus rotating the imaging array 38, to facilitate imaging even after the needle and tines have been deployed.

While the above is a complete description of the preferred embodiments of the invention, various alternatives, modifications, and equivalents may be used. Therefore, the above description should not be taken as limiting the scope of the invention which is defined by the appended claims.

## Claims

1. An imaging and therapeutic delivery system comprising:
a straight shaft (12) having a distal end and a proximal end;
a needle (70) reciprocatably coupled to the shaft so that a tissue-penetrating tip on the needle can be distally advanced from the shaft along an axial path;
a tip (18) pivotally attached to the distal end of the shaft and movable between a position parallel to the axial path and a position at an acute or right angle relative to the axial path, wherein the tip (18) includes a stop structure (19) disposed thereon so that the stop structure (19) prevents advancement of the needle (70) when the tip (18) is parallel to the axial path, and wherein deployment of the tip (18) moves the stop structure (19) out of the advancement path of the needle (70); and
an ultrasonic imaging array (38) carried by the pivotally attached tip (18), wherein the tip can be oriented to align a field of view of the imaging array with the needle (70) as the needle is advanced along the axial path so as to sweep the ultrasound field of view relative to the needle and anatomy to be imaged,
the system further comprising a plurality of tines (82) carried by the needle (70), wherein the tines are reciprocatably attached to the needle.

2. A system as in claim 1, wherein the tip (18) is offset from the axial path of the needle (70).

3. A system as in claim 1 or 2, further comprising an imaging core which includes the imaging array (38) near a distal end thereof, wherein the imaging core is removably positionable in the straight shaft (12) so that the imaging array extends into the pivotally attached tip (18).

4. A system as in claim 3, wherein the straight shaft (12) is relatively rigid and the imaging core is flexible, wherein the imaging core can bend at the point where the tip is pivotally attached to the shaft.

5. A system as in claim 3, wherein the straight shaft (12) is relatively rigid and the imaging core is hinged at the point where the tip is pivotally attached to the shaft.

6. A system as in claim 1, wherein the ultrasonic imaging array is configured to sweep the ultrasound field of view relative to the anatomy, needle (70) and plurality of tines (82).

7. A system as in any of the preceding claims, wherein the tines (82) are arranged to radially diverge from the needle (70) as they are advanced distally.

8. A system as in claim 7, wherein at least one additional tine is arranged to advance axially from the needle (70).

9. A system as in any of the preceding claims, wherein the needle (70) is electrically non-conductive and the tines (82) are electrically conductive.

10. A system as in any one of the preceding claims, wherein rotation of said ultrasonic imaging array is independent of movement of said needle (70).

11. A system as in any one of the preceding claims, wherein the needle (70) is reciprocatably deployed in the shaft (12).

## Patentansprüche

1. Ein Bildgebungs- und therapeutisches Zuführsystem, das Folgendes beinhaltet:
einen geraden Schaft (12) mit einem distalen Ende und einem proximalen Ende;
eine Nadel (70), die hin- und herbewegbar mit dem Schaft gekoppelt ist, sodass eine gewebepenetrierende Spitze auf der Nadel entlang eines axialen Weges von dem Schaft distal vorgeschoben werden kann;
eine Spitze (18), die schwenkbar an dem distalen Ende des Schafts befestigt ist und zwischen einer Position, die parallel zu dem axialen Weg ist, und einer Position in einem spitzen oder rechten Winkel relativ zu dem axialen Weg beweglich ist, wobei die Spitze (18) eine Anschlagstruktur (19) umfasst, die so darauf angeordnet ist, dass die Anschlagstruktur (19) ein Vorschieben der Nadel (70) verhindert, wenn die Spitze (18) parallel zu dem axialen Weg ist, und wobei das Einsetzen der Spitze (18) die Anschlagstruktur (19) aus dem Vorschubweg der Nadel (70) bewegt; und
eine Ultraschallbildgebungsanordnung (38), die von der schwenkbar befestigten Spitze (18) getragen wird, wobei die Spitze so orientiert sein kann, dass sie ein Sehfeld der Bildgebungsanordnung mit der Nadel (70) ausrichtet, während die Nadel entlang des axialen Weges vorgeschoben wird, um somit das Ultraschallsehfeld relativ zu der Nadel und der abzubildenden Anatomie zu durchsuchen, wobei das System ferner eine Vielzahl von Zinken (82), die von der Nadel (70) getragen werden, beinhaltet, wobei die Zinken hin- und herbewegbar an der Nadel befestigt sind.

2. System nach Anspruch 1, wobei die Spitze (18) zu dem axialen Weg der Nadel (70) versetzt ist.

3. System nach Anspruch 1 oder 2, das ferner einen Bildgebungskern beinhaltet, der die Bildgebungsanordnung (38) in der Nähe eines distalen Endes davon umfasst, wobei der Bildgebungskern entfernbar in dem geraden Schaft (12) positioniert werden kann, sodass sich die Bildgebungsanordnung in die schwenkbar befestigte Spitze (18) hinein erstreckt.

4. System nach Anspruch 3, wobei der gerade Schaft (12) relativ starr ist und der Bildgebungskern flexibel ist, wobei sich der Bildgebungskern an dem Punkt biegen kann, wo die Spitze schwenkbar an dem Schaft befestigt ist.

5. System nach Anspruch 3, wobei der gerade Schaft (12) relativ starr ist und der Bildgebungskern an dem Punkt, wo die Spitze schwenkbar an dem Schaft befestigt ist, drehbar angebracht ist.

6. System nach Anspruch 1, wobei die Ultraschallbildgebungsanordnung dazu ausgelegt ist, das Ultraschallsehfeld relativ zu der Anatomie, der Nadel (70) und der Vielzahl von Zinken (82) zu durchsuchen.

7. System nach einem der vorhergehenden Ansprüche, wobei die Zinken (82) so angeordnet sind, dass sie radial von der Nadel (70) auseinanderlaufen, während sie distal vorgeschoben werden.

8. System nach Anspruch 7, wobei mindestens eine zusätzliche Zinke so angeordnet ist, dass sie axial von der Nadel (70) vorgeschoben wird.

9. System nach einem der vorhergehenden Ansprüche, wobei die Nadel (70) elektrisch nicht leitend ist und die Zinken (82) elektrisch leitend sind.

10. System nach einem der vorhergehenden Ansprüche, wobei die Rotation der Ultraschallbildgebungsanordnung unabhängig von der Bewegung der Nadel (70) ist.

11. System nach einem der vorhergehenden Ansprüche, wobei die Nadel (70) hin- und herbewegbar in dem Schaft (12) eingesetzt wird.

## Revendications

1. Système d'imagerie et de distribution de traitement comprenant :
une tige droite (12) ayant une extrémité distale et une extrémité proximale ;
une aiguille (70) couplée, de manière à pouvoir se déplacer en va-et-vient, à la tige de sorte qu'une pointe pénétrant le tissu sur l'aiguille peut être avancée de manière distale depuis la tige le long de l'axe d'un trajet axial ;
une pointe (18) attachée de manière pivotante à l'extrémité distale de la tige et pouvant être déplacée entre une position parallèle au trajet axial et une position à angle aigu ou droit par rapport au trajet axial, dans lequel la pointe (18) inclut une structure d'arrêt (19) disposée sur celle-ci de sorte que la structure d'arrêt (19) empêche l'avancée de l'aiguille (70) quand la pointe (18) est parallèle au trajet axial, et dans lequel le déploiement de la pointe (18) déplace la structure d'arrêt (19) hors du trajet d'avancée de l'aiguille (70) ; et
un réseau d'imagerie ultrasonore (38) porté par la pointe attachée de manière pivotante (18), dans lequel la pointe peut être orientée pour aligner un champ de vision du réseau d'imagerie avec l'aiguille (70) quand l'aiguille est avancée le long du trajet axial de manière à balayer le champ de vision ultrasonore par rapport à l'aiguille et l'anatomie à imager,
le système comprenant en outre une pluralité de dents (82) portées par l'aiguille (70), dans lequel les dents sont attachées à l'aiguille de manière à pouvoir se déplacer en va-et-vient.

2. Système selon la revendication 1, dans lequel la pointe (18) est décalée par rapport au trajet axial de l'aiguille (70).

3. Système selon la revendication 1 ou 2, comprenant en outre un noyau d'imagerie qui inclut le réseau d'imagerie (38) près d'une extrémité distale de celui-ci, dans lequel le noyau d'imagerie peut être positionné de manière amovible dans la tige droite (12) de sorte que le réseau d'imagerie s'étend jusque dans la pointe attachée de manière pivotante (18).

4. Système selon la revendication 3, dans lequel la tige droite (12) est relativement rigide et le noyau d'imagerie est flexible, dans lequel le noyau d'imagerie peut se plier au niveau du point où la pointe est attachée de manière pivotante à la tige.

5. Système selon la revendication 3, dans lequel la tige droite (12) est relativement rigide et le noyau d'imagerie est articulé au niveau du point où la pointe est attachée de manière pivotante à la tige.

6. Système selon la revendication 1, dans lequel le réseau d'imagerie ultrasonore est configuré pour balayer le champ de vision ultrasonore par rapport à l'anatomie, l'aiguille (70) et la pluralité de dents (82).

7. Système selon l'une quelconque des revendications précédentes, dans lequel les dents (82) sont agencées pour être déviées de manière radiale par rapport à l'aiguille (70) quand elles sont avancées de manière distale.

8. Système selon la revendication 7, dans lequel au moins une dent supplémentaire est agencée pour être avancée de manière axiale depuis l'aiguille (70).

9. Système selon l'une quelconque des revendications précédentes, dans lequel l'aiguille (70) est non conductrice électriquement et les dents (82) sont conductrices électriquement.

10. Système selon l'une quelconque des revendications précédentes, dans lequel la rotation dudit réseau d'imagerie ultrasonore est indépendante du mouvement de ladite aiguille (70).

11. Système selon l'une quelconque des revendications précédentes, dans lequel l'aiguille (70) est déployée de manière à pouvoir se déplacer en va-et-vient dans la tige (12) .
